# EUROPEAN PATENT APPLICATION

(11) **EP 2 520 282 A2**
(43) Date of publication of application: **07.11.2012**
(21) Application number: 12166417.1
(22) Date of filing: 02.05.2012
(51) Int. Cl.: A61K 9/14, B65B 3/00

(54) **Vials of vancomycin vhloridrate with improved stability**

(30) Priority: 03.05.2011 IT LO20110001
(71) Applicant: Fisiopharma Srl, 84020 Palomonte (SA) (IT)
(72) Inventor: Ardia, Rosario, 84025 Eboli (SA) (IT); Magliano, Brunilde, 84126 Salerno (IT)
(74) Representative: Serravalle, Marco

(57) **Abstract**

The present invention is directed to vials containing vancomycin characterized in that the gas phase of the vials contains an amount of oxygen equal to or lower than 5 wt %. Furthermore, the invention is also directed to a method for the preparation of the vials comprising the following steps: 1) flowing an inert gas in the empty vial, 2) adding vancomycin to the vial, and 3) flowing the inert gas in the vial filled with vancomycin at a lower flow rate than in step 1).

## Description

### Field of the invention

. The present invention is directed to sterile vials of vancomycin chlorohydrate presenting improved storage stability. The vials are characterized by the presence of an inert atmosphere wherein the amount of oxygen is equal to or lower than 5 wt%.

### Background of the invention

. Vancomycin is a tricyclic glycopeptide antibiotic derived from Amycolaptopsis orientalis. It is used in the prophylaxis and treatment of infection caused by Gram-positive bacteria. Because of its low oral bioavalability, it is administered mainly by intravenous route. Vancomycin needs to be administered slowly and it is usually prepared as a dilute solution (e.g. 5 mg/mL). While other drugs are commercially available as a concentrate solution, which is then diluted to the desired concentration just before administration, vancomycin is commercially available as a vial of powdered vancomycin. In fact, vancomycin is highly unstable and would have a very limited storage time in solution.

. Powdered drugs have normally a much longer storage time than solutions; however, in the case of vancomycin, after 2 years of storage, the powder becomes colored (pink and then brown) and the drug is no more within the requirement of European Pharmacopoeia. This fact leads to a considerable amount of product which cannot be used and, apart from the financial loss for the producer, we have also environmental problems due to the need of safely disposing the off-specs drug.

. Purification of vancomycin is a complicated task and very high levels of purity are not easily achieved. This fact is confirmed by the European Pharmacopoeia that requires a minimum title of vancomycin B of 93 wt %. Another important requirement of the European Pharmacopoeia is that the absorbance at 450 nm of a solution of 2.5 g of vancomycin in 25 ml of water must be lower than 0.1. This implies that the powder must be white or almost white.

. In the past, several attempts have been made to increase storage stability of vancomycin, mostly by increasing the title of the drug by improving the purification process.

. EP 132 117 (Smithkline Beckman Corporation) discloses an affinity chromatography process for the preparation of purified vancomycin class antibiotics which comprises: (1) the contacting of an impure solution containing the antibiotic with a solid carrier matrix to which immobilizing ligands selected from -D-alanyl-D-alanine or -X-D-alanyl-D-alanine, wherein X is an amino acid radical, have been attached; (2) thereby forming a sorption complex between the antibiotic and the immobilizing ligand; (3) removing the impurities from the matrix and complex attached thereto; and (4) dissociating the complex to isolate and recover the purified antibiotic.

. WO 2007/068644 (Pharmatex Italia srl) discloses a process for the purification of macrolide antibiotics, e.g. vancomycin, performed by dissolving the macrolide antibiotic in water and subjecting the solution to ultrafiltration with a membrane having nominal retention lower than 30,000 Da.

. All these attempts to improve storage stability of vancomycin HCl produced limited effect and pharmaceutical industries are still faced with relevant amounts of vancomycin which have to be withdrawn due to non-compliance with the requirements of European Pharmacopoeia.

### Summary of the invention

. The present invention concerns the finding that a relevant improvement in storage stability of vancomycin can be achieved by storing the antibiotic in powder form in vials under an inert gas atmosphere containing equal to or less than 5.0 wt % of oxygen, preferably equal to or lower than 2 wt %, most preferably equal to or lower than 1 wt %.

. In another embodiment, the present invention is also directed to a method for preparing vials containing vancomycin, which method comprises the following steps: 1) flowing an inert gas in the empty vial, 2) quickly adding vancomycin to the vial and 3) flowing the inert gas in the vial filled with vancomycin at a lower flow rate than in step 1), to avoid that part of the powder is entrained in the gas flow and washed away from the vial. By the use of this method, it is possible to achieve residual oxygen level equal to or lower than 5 wt %.

### Detailed description of the invention

. Fig. 1 represents a typical vial for use in the present invention. The vial can be made of glass, plastic or any other suitable material. More preferably the vial is a glass vial.

. In a first embodiment the invention relates to vials containing vancomycin and wherein the gas present in the vial presents an oxygen concentration equal to or lower than 5.0 wt %, preferably lower than 1.0 wt %. In a preferred embodiment the vials contain from 0.1 to 10.0 g, more preferably from 0.5 to 1.0 g of vancomycin chlorohydrate.

. The gas present in the vial can be any inert gas, such as nitrogen, helium, argon, carbon dioxide. It has been found that the use of different inert gases does not produce major changes in stability. The most preferred inert gas is nitrogen.

. The invention is also directed to a new method for preparing vials containing from 0.1 to 10.0 g of vancomycin powder, which method allows the achievement of the reduced oxygen content required in order to improve storage stability.

. In fact, conventional machines for preparing vials containing a powdered drug, even when provided of a step wherein an inert gas is flown through the vial, produce vials containing oxygen levels much higher than 5 wt %.

. Thus, the present invention is also directed to a method for preparing vials containing vancomycin, which method comprises the following steps: 1) flowing an inert gas in the empty vial, 2) adding vancomycin to the vial and 3) flowing the inert gas in the vial filled with vancomycin at a lower flow rate than in step 1), to avoid that part of the powder is entrained in the gas flow and washed away from the vial. By the use of this method, it is possible to achieve residual oxygen level equal to or lower than 5.0 wt %.

. With reference to the vials of fig.1, the gas injectors which flow the inert gas before and after vancomycin addition, enter the vial for a depth of about 5 mm in a central position.

. The gas flow before vancomycin addition is preferably comprised between 10 and 20 NL/min (normal liter per minute), while the gas flow after vancomycin addition is preferably kept between 4 and 8 NL/min. The gas flow is maintained in the vial for a time comprised between 1 s and 20 s, preferably from 2 s to 10 s, both before and after vancomycin addition.

### Experimental

. The vials were first filled in by using a conventional machine which foresees a gas bubbling after the powder has been introduced in the vial. However, even when increasing the time of flux of the inert-gas, the residual oxygen in the vial was always higher than 5 wt %. Consequently, a pre-flux of inert gas has been added and in this way it has been possible to achieve oxygen levels up to less than 1 wt %.

. Process conditions have been varied and fixed in three different ways which allowed the achievement of three different levels of residual oxygen: lower than 1 wt%, from 1 to 2 wt% and from 2 to 5 wt %. Furthermore, vials were prepared with no inert gas flux, according to the prior art.

. A first set of experiment was performed to evaluate the influence of the percentage of oxygen on vancomycin stability at 40°C. This is an accelerated ageing experiment to have a quicker response than obtained at the standard storage temperature of 25°C. From these data it is possible to see that decreasing the amount of oxygen increases the stability of vancomycin. While storage in air resulted in a stability of less than 1 month, reducing the amount of oxygen at a level comprised between 2 and 5 wt% produced a stability at 40°C of less than 2 months, 1-2 wt % oxygen resulted in a stability of less than 5 months, while an oxygen level lower than 1 wt % produced a stability of at least 6 months (Table I).

. The parameters which were checked on the samples, according to the European Pharmacopoeia 7.0, were the following: color (white or almost white), solubility (freely soluble in water and slightly soluble in alcohol), appearance of the solution (clear), humidity (<5%), absorbance at 450 nm (<0.10), pH (2.50-4.50), percentage vancomycin (>93%), single impurity (<4.0 %) total impurities (<7.0%), potency (>1050 IU/mg). Table I indicates if the sample were OK and if not which parameters failed.

. Since the objective of the invention was to increase the storage stability to at least 3 years, it was considered if a reduction in oxygen content to a level equal to or lower than 5 wt % would have been sufficient to achieve the objective. The study conducted on several samples shows that when oxygen is equal to or lower than 5 wt %, stability is at least 3 years.

. The data in table 2 show that oxygen content comprised between 2 and 5 wt % results in a stability of at least 3 years.

**Table I**

| Accelerated ageing at 40 °C | | | | | | | |
|---|---|---|---|---|---|---|---|
| Sample | T° | 1 month | 2 months | 3 months | 4 months | 5 months | 6 months |
| Air | OK | Off specs^{a} | | | | | |
| N₂ av. O₂ 3.4% | OK | OK | Off specs^{b} | | | | |
| N₂ av. O₂ 1.6% | OK | OK | OK | OK | OK | Off specs^{c} | |
| He av.O₂ 1.6% | OK | OK | OK | OK | OK | Off specs^{d} | |
| N₂ av. O₂ 0.9% | OK | OK | OK | OK | OK | OK | OK |
| CO₂ av.O₂ 0.9% | OK | OK | OK | OK | OK | OK | OK |
| Ar av. O₂ 0.9% | OK | OK | OK | OK | OK | OK | OK |

| | | | | | | | |
|---|---|---|---|---|---|---|---|
| a % vancomycin, single and total impurities, absorbance, potency b % vancomycin, single and total impurities c % vancomycin, absorbance, potency d % vancomycin, single and total impurities, absorbance | | | | | | | |

**Table II**

| Ageing at 25 °C - Parameters after 36 months | | | | | |
|---|---|---|---|---|---|
| Sample | Absorbance AU | % Vanc. | Single imp. % | Total Imp. % | Potency IU/mg |
| A | 0.05 | 95.0 | 0.9 | 4.7 | 1065 |
| B | 0.05 | 94.3 | 1.1 | 5.5 | 1080 |
| C | 0.06 | 94.4 | 1.0 | 4.6 | 1109 |
| D | 0.05 | 93.0 | 1.3 | 7.0 | 1108 |
| E | 0.07 | 94.9 | 1.1 | 3.9 | 1112 |
| F | 0.06 | 94.4 | 1.0 | 4.6 | 1109 |

| | | | | | |
|---|---|---|---|---|---|
| Samples A, B and C contained 1000 mg of vancomycin, samples D,E and F 500 mg | | | | | |

. Samples A-F were prepared by fixing the nitrogen flux and the time of flux in order to obtain an amount of oxygen in the vials comprised between 2 and 5 wt %. The results show that in all cases all measured parameters of vancomycin were still within the specifications of the European Pharmacopoeia 7.0 after 3 years. It is known that vials filled without inert gas last only 2 years.

## Claims

1. Vials containing powdered vancomycin in an amount of at least 93 wt % **characterized in that** the gas phase of the vials contains an amount of oxygen equal to or lower than 5 wt %.

2. Vials according to claim 1 wherein the amount of oxygen is equal to or lower than 2 wt %.

3. Vials according to claim 2 wherein the amount of oxygen is equal to or lower than 1 wt %.

4. Vials according to claims 1-3 wherein the amount of vancomycin is comprised between 0.100 and 10.0 g.

5. Method for the preparation of the vials according to claims 1-4 comprising the following steps:
1) flowing an inert gas in the empty vial,
2) adding vancomycin to the vial, and
3) flowing the inert gas in the vial filled with vancomycin at a lower flow rate than in step 1).

6. Method according to claim 5 wherein the inert gas is selected form nitrogen, helium, argon and carbon dioxide.

7. Method according to claims 5-6 wherein the inert gas flow rate in step 1) is comprised between 10 and 20 NL/min.

8. Method according to claims 5-7 wherein the inert gas flow rate in step 3) is comprised between 4 and 8 NL/min.

9. Method according to claims 5-8 wherein the inert gas flow in each of steps 1) and 3) is maintained for a time comprised between 1 and 20 s.
